Europäisches Patentamt

European Patent Office

Office européen des brevets

(11) Publication number: **0 421 382 B1**

# EUROPEAN PATENT SPECIFICATION

(45) Date of publication of patent specification: **31.08.94**　(51) Int. Cl.5: **C12P 21/08**, A61K 39/395, C12N 5/12

(21) Application number: **90118962.1**

(22) Date of filing: **04.10.90**

(54) Monoclonal antibody to exotoxin A of pseudomonas aeruginosa.

(30) Priority: **04.10.89 JP 260827/89**
**25.09.90 JP 256450/90**

(43) Date of publication of application:
**10.04.91 Bulletin  91/15**

(45) Publication of the grant of the patent:
**31.08.94 Bulletin  94/35**

(84) Designated Contracting States:
**AT BE CH DE DK FR GB IT LI NL SE**

(56) References cited:
EP-A- 0 305 960
EP-A- 0 322 762

(73) Proprietor: **SUMITOMO PHARMACEUTICALS COMPANY, LIMITED**
**2-8, Doshomachi 2-chome,**
**Chuo-ku**
**Osaka 541 (JP)**

(72) Inventor: **Ohtsuka, Hiroshi**
**6-14-D1606, Minatojimanaka-machi,**
**Chuo-ku**
**Kobe-shi, Hyogo-ken (JP)**
Inventor: **Ochi, Hiroshi**
**2-11-8-110, Sonehigashi-machi**

**Toyonaka-shi, Osaka-fu (JP)**
Inventor: **Noguchi, Hiroshi**
**4-4-153, Seiwadainishi**
**Kawanishi-shi, Hyogo-ken (JP)**
Inventor: **Yokota, Shinichi**
**2-14-7, Mefu**
**Takarazuka-shi, Hyogo-ken (JP)**
Inventor: **Kohzuki, Tsuneo**
**2-25-8, Yuyamadai**
**Kawanishi-shi, Hyogo-ken (JP)**
Inventor: **Irie, Kenji**
**2-3-21-108, Ohgi,**
**Higashinada-ku**
**Kobe-shi, Hyogo-ken (JP)**

(74) Representative: **VOSSIUS & PARTNER**
**Postfach 86 07 67**
**D-81634 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

The present invention relates to a human monoclonal antibody to exotoxin A of Pseudomonas aeruginosa (hereinafter referred to as "P. aeruginosa"), a cell line producing said monoclonal antibody, and a prophylactic and therapeutic formulation containing said monoclonal antibody as an essential component. The antibody of the present invention has a strong activity in neutralizing cytotoxicity caused by exotoxin A, and accordingly, it is useful for prevention and treatment of infectious diseases caused by P. aeruginosa.

The kinds of bacteria causing infectious diseases, i.e., prophlogistic bacteria have changed with development and change of clinically used antibiotics. As the result, infectious diseases caused by bacteria which used to show only low pathogenicity or virulence have increased. Thus, P. aeruginosa is currently one of the major pathogenic bacteria causing infectious diseases, of which serious symptoms often lead patients to death, particularly when their immunity is low due to continuous administration of immunosuppressants, suffering from cancer or burn, new born, or the like. Pathogenic substances of P. aeruginosa include endotoxins which generate with the growth of the cell, exotoxins and exoenzymes produced by the cell. Above all, exotoxin A is commonly produced by most of various P. aeruginosa strains clinically isolated, and lethal to host animals.

Administration of immunoglobulin, i.e., so called "antibody therapy" has been attracting the attention of physician, which therapy prevents and treats microbisms by neutralizing or inhibiting toxins or enzymes derived from microorganisms. This therapy can be performed in association with, or in place of, chemotherapy. A serum of high antibody titer can be obtained by active immunization of animals such as horse or rabbit, and antibody therapy can be made by administration of such serum. In fact, its remarkable therapeutic effect was proved on experimental infections using various animals.

It is known from the cases of diphtheria toxin and viper toxin that antibody therapy using sera originated from animals is quite effective even on human beings.

Conventional human immunoglobulin preparations are manufactured by collecting blood from healthy persons or bacteria-infected patients, subjecting the blood to fractionation to obtain an immunoglobulin fraction, purifying the immunoglobulin fraction, and eliminating agglutinating materials therefrom by addition of ethylene glycol, treatment with protease, sulfonization, DEAE-column chromatography, etc., followed by formulation of the resulting product into intramuscularly or intravenously injectable preparations. These preparations have several drawbacks. One of such drawbacks is that their antibody titer against bacteria, or toxins or enzymes derived from the bacteria, is so low that a sufficient therapeutic effect can not necessarily be produced. Another drawback is that their stable supply with a high antibody titer in a large amount is difficult, because they are manufactured using the blood collected from healthy persons or bacteria-infected patients and the constant and continuous obtainment of sera having a high antibody titer is quite hard. A further drawback is that they may be contaminated with hepatitis virus (e.g. HB virus), or unidentified pathogens such as Adult T cell leukemia virus (ATLV), Human immunodeficiency virus (HIV), or non A - non B hepatitis virus, because the blood as the starting material is obtained from a number of unknown persons. It is thus highly desired to develop immunoglobulin preparations which have higher antibody titer against bacteria, or toxins or enzymes derived from the bacteria, in particular against exotoxins of P. aeruginosa, and therefore, show remarkable therapeutic effect on microbisms, and yet which is safe to use.

Exotoxin A, one of exotoxins produced by P. aeruginosa, is a protein consisting of 613 amino acids. The exotoxin A of P. aeruginosa exhibits ADP ribosyltransferase activity. The protein binds to receptors existing on the surface of animal cells, penetrates into the cells, and inhibits protein-synthesis of the cells, thereby exerting its toxicity.

Monoclonal antibodies to exotoxin A of P. aeruginosa have been reported by D.R.Galloway et al., Infection and Immunity, vol.44, pp.262-267, 1984, "Production and characterization of monoclonal antibodies to exotoxin A from Pseudomonas aeruginosa"; Noguchi et al, Japanese Patent Publication (Kokai) No. 204200/1986; J.M. Larrick et al., United State Patent No. 4,677,070, January 30, 1987, "Pseudomonas aeruginosa exotoxin A antibodies, their preparation and use"; A.W. Anthony et al., PCT/US86/01204, "Protective human monoclonal antibodies to Pseudomonas aeruginosa exotoxin A; J.K. Chia et al., Infection and Immunity, vol.52, pp756-762, 1986, "Functionally distinct monoclonal antibodies reactive with enzymatically active and binding domain of Pseudomonas aeruginosa; and Iwasa et al., Japanese Patent Publication (Kokai) No. 63374/1989 (equivalent to EP-A-0 305 960) and EP-A-322 762. However, none of the antibodies disclosed in the prior art has not practically used in antibody therapy. Thus, development of human monoclonal antibody to exotoxin A of P. aeruginosa, which shows stronger toxin-neutralizing effect and therapeutic effect, has been still desired.

In general, it is well known that an antitoxic antiserum can neutralize the toxin. The molecule of a given toxin has many epitopes. Among them, epitopes which are involved in expression of toxicity are important

2

as targets of neutralizing antibodies. Such important epitopes include a binding site to target cells and a moiety responsible for enzymatic activity of the toxin. Antitoxic antiserum comprises polyclonal antibodies to various epitopes of a toxin. Some of them bind to epitopes which are important for the expression of toxicity, thereby inhibiting the expression and exerting neutralizing activity. To the contrary, monoclonal antibody binds to a single epitope. Accordingly, all of the monoclonal antibodies to a toxin do not have the neutralizing activity. Only antibodies which are directed to important epitopes associated with the expression of toxicity exhibit the neutralizing activity. Other antibodies not related to the expression have no activity.

There is available a detailed report on the relationship between epitopes of tetanus toxin recognized by antibodies and toxicity-neutralizing activity of the antibodies. Thus, Volk et al. obtained 57 monoclonal antibodies toward 20 or more epitopes of tetanus toxin. Their experimental study revealed that nine of them showed a strong neutralizing activity on the tetanus toxin, and that they all recognized particular epitopes. The study further showed that there was a significant correlation between toxicity-neutralizing activity of an antibody and its epitope to which the antibody binds, and that antibodies specific to particular epitopes show neutralizing activity, while other antibodies directed to other epitopes do not have such activity even though they are capable of binding to the tetanus toxin.

As can be seen from the above, therapeutically-effective monoclonal antibody to exotoxin A of P. aeruginosa must meet the requirements that it binds to particular epitopes on the molecule of exotoxin A and inhibits the function of the exotoxin A. Thus, all of the monoclonal antibodies to exotoxin A of P. aeruginosa are not therapeutically effective, and only some of them are clinically useful.

It is not foreseeable which antibody or antibodies are clinically effective and useful. It is thus very important to obtain monoclonal antibody which is practically effective and useful, and identify particular epitope recognized by the antibody.

Important factors which determine usefulness of an antibody as a therapeutic agent include its binding constant and pH-dependency as well as its epitope. The pH-dependency has been illustrated in the case of diphtheria toxin, but there is no report on exotoxin A of P. aeruginosa.

In view of the situation as mentioned above, the inventors of the present invention have prepared a lot of human monoclonal antibodies to exotoxin A of P. aeruginosa, and investigated them in order to select improved monoclonal antibody. Thus, extensive studies on the therapeutic effect and physicochemical, immunochemical and biological properties of the antibodies allowed the inventors to select a monoclonal antibody which has a potent neutralizing activity against exotoxin A of P. aeruginosa and which is therapeutically valuable in treating P. aeruginosa infections when used alone or together with antibiotics. The present invention is based on this finding.

Thus, the present invention provides a human monoclonal antibody to exotoxin A of P. aeruginosa which shows a potent cytotoxicity-neutralizing activity and which is therapeutically effective for treating P. aeruginosa infections. This invention also provides a cell line producing said monoclonal antibody, a high titer immunological preparation containing said monoclonal antibody, and methods of the preparation thereof.

The invention further provides the method for treating P. aeruginosa infections by administering said preparation containing the nonoclonal antibody to exotoxin A of P. aeruginosa.

The monoclonal antibody of the present invention is characterized in that it has immunochemical, biological, and physicochemical properties listed below.

1. The monoclonal antibody specifically binds to exotoxin A of P. aeruginosa.

2. One molecule of the monoclonal antibody neutralizes cytotoxicity caused by more than one molecular of exotoxin A of P. aeruginosa in BALL 1 cell culture.

3. $4 \times 10^{-12}$ mole (1 $\mu$g) of the monoclonal antibody parenterally administered to ICR-slc mice (4-week, male, 23-24 g) neutralizes lethal toxicity caused by $15 \times 10^{-12}$ mole (0.6 $\mu$g) of exotoxin A of P. aeruginosa and keeps more than 50% of mice alive.

4. The monoclonal antibody alone is therapeutically effective for the treatment of experimentally-induced P. aeruginosa infections which release exotoxin A.

5. The monoclonal antibody shows synergistic effects in the treatment of P. aeruginosa infections involving exotoxin A, when used together with antibiotics.

6. The monoclonal antibody exhibits maximum avidity at pH higher than 5, and thus binding of the monoclonal antibody to the exotoxin A is kept stable at pH 3.0.

7. The epitope of the monoclonal antibody exists in 591-613 amino acid sequence

(Glu-Gln-Ala-Ile-Ser-Ala-Leu-

Pro-Asp-Tyr-Ala-Ser-Gln-Pro-Gly-Lys-Pro-Pro-Arg-Glu-Asp-Leu-

Lys)

of exotoxin A of P. aeruginosa, which residues are characterized in that they contain Tyr residue.

8. The monoclonal antibody belongs to IgG 3 class.

Specific example of the antibody of the present invention which meets the above requirements is human monoclonal antibody HI-1A4. The antibody may be obtained from either a Epstein-Barr Virus (EBV)-transformed cell or a hybridoma which is prepared in the following manner.

EBV is used to infect B lymphocytes derived from donors who have been naturally sensitized against exotoxin A through P. aeruginosa infection and have high serum-level of antibody titer, thereby transformants which are capable of growing continuously being obtained. Addition of an immunosuppressive such as cyclosporin A in the above step is desirable in order to suppress the activity of cytotoxic T cells.

A clone carrying the afore-mentioned characteristic properties is selected from the transformed cells, or cloned transformed cells, and the selected clone is allowed to continuously grow in vitro. Thus, a cell line capable of continuously growing and producing the desired antibody is established. Selection of the clone includes conventional immunological assay such as radioimmuno-assay (RIA) in a solid or liquid phase, or enzyme linked immunosorbent assay (ELISA), and conventional biological assay such as in vitro exotoxin A-neutralizing test (e.g., cytotoxicity-neutralizingtest). The cloning may be done by any of limiting dilution method, soft agar method, and single cell manipulation method. Starting from monoclone, there is obtained an established cell line, so-called B lymphoblastoid cell, which exhibits rapid growth and produces an antibody reactive to exotoxin A of P. aeruginosa. The use of a protective cell and addition of a lymphokine like human IL-6 can improve the cloning efficiency.

The established transformed cells obtained above, which has been prepared through transformation by EBV and which produces the specific antibody, can be used to prepare a hybridoma cell line capable of rapidly growing and continuously producing abundant specific antibody to exotoxin A of P. aeruginosa, by subjecting the transformed cells to a cell fusion with myeloma cell. Cultivation of the established hybridoma cell line gives abundant antibodies secreted in the culture medium.

Preparation of the above-mentioned EBV-transformant and the hybridoma is further described in more detail below.

As the human B lymphocytes, there may be used human B lymphocytes producing an antibody to exotoxin A of P. aeruginosa, which can be separated from peripheral blood by centrifugation using a lymphocyte separation liquid such as Lymphoprep[R] or Mono-Poly Resolving medium[R] (Flow Lab.). There may be also used B lymphocytes originated from tissues or organs (e.g., lymphnode, spleen) extracted for the purpose of diagnosis or therapy of diseases, umbilical cord blood, or the like. It is desirable to obtain the cells from patients who were infected with P. aeruginosa in the past and whose cells were sensitized with P. aeruginosa. Pertinent persons, from whom the cells may be obtained, can be chosen by pre-measurement of the antibody titer in their sera.

Human B lymphocytes have such characteristic properties that they carry antibody molecules on their cell surfaces and can secrete only a limited amount of antibody during a limited time, but they cannot grow unlimitedly.

For changing the human B lymphocytes to continuously and unlimitedly proliferable cell lines, two methods can be employed in the present invention.

The first method comprises infecting the sensitized B lymphocyte with EBV, which has been prepared from marmoset cell B95-8, by shake culture. Thus, the lymphocytes are plated on a 96 well microplate at a rate of 0.5 - 3 x $10^4$ cells/well. By the use of a preselected conventional medium such as RPIM1640 medium, Dulbecco's medium, or Iscove's medium containing 2-20% (v/v) fetal bovine serum, the lymphocytes are cultured at 32-37°C for 2-5 weeks in the presence of 5-10% $CO_2$ to obtain transformed cells. During the culture, half of the medium is changed every 2 or 4 days. If necessary, 1-2 $\mu$g/ml of cyclosporin A, antibiotics, or synthetic antimicrobes which prevent the medium from mycoplasma infections, and/or mouse peritoneal cells, as feeder cells, may be added to the medium. Ten days after infection, cell populations of 20-200 transformed cells are optical-microscopically observed and can be distinguished from non-transformed cells. The culture supernatants from the wells which contain well-grown transformed cells are tested for specific antibody titer by ELISA. As for the supernatants from positive wells with respect to the antibody titer, there are selected antibodies which can bind intensively to exotoxin A of P. aeruginosa during competitive reaction in ELISA. Further selection and cloning are made for antibodies which intensively neutralize in vitro the cytotoxicity of exotoxin A of P. aeruginosa. The cloning is conducted by the use of limiting dilution method in which the cluster of transformed cells is scattered by repeated inhaling and exhaling of a solution containing the cluster with a pipette, diluted with the culture medium to an appropriate concentration, and planted and cultured on a 96 well microplate at a rate of 0.5 - 100 cells/well. In the cloning, it is preferred to use mouse peritoneal cells, human umbilical cord blood lymphocytes, or X-ray treated mouse spleen cells as protective cells. Cloning efficiency may be increased by addition of lymphokine such as IL-6. Antibody titer of culture supernatant of cloned cell lines is measured by ELISA, and clones producing abundant specific antibody are selected. The cloning process and the selection of high titer antibody-producing clones are repeated two or three times, and there is established a transformed cell line which shows rapid growth, and abundant and steady production of specific antibody.

The second method comprises a cell fusion of the transformant established in the first method mentioned above with a myeloma cell. Myeloma cells employable in the cell fusion include hypoxanthin-guanine phosphoribosyl transferase (HPRT)-deficient mouse myeloma cells, such as P3-X63-Ag8-U1 (P3U1;ATCC CRL1597), P3-NS-1/1-Ag4-1 ( NS-1;ATCC TIB 18), P3-X63-Ag8-653 (653;ATCC CRL1580), SP2/0-Ag14 (SP2/0;ATCC CRL8287), SHM-D33 (ATCC CRL1668), and the like. Other human immunoglobulin-nonproducing or -nonsecreting cells, or cells conventionally employed in the art, may also be used.

The cell fusion techniques include HVJ method (Sendai virus is employed), polyethylene glycol (PEG) method, and electric fusion method. Specific antibody-producing cells are selected after measuring by ELISA an antibody titer of hybridoma culture medium. The cloning is repeated two or three times by means of limiting dilution method or soft agar method. There is thus obtained EBV-free established cell line capable of growing rapidly and producing abundant specific antibody.

The thus established cell lines obtained from antigen-sensitized human B lymphocytes according to the above-mentioned "EBV transformation method" and "EBV-hybridoma method" are characterized in that they are capable of continuously growing and steadily producing abundant specific antibody.

Chemical treatment (e.g., DNA mutagenic agent) or physical treatment (e.g., UV radiation) of the EBV-transformed cell line or hybridoma obtained above can provide descendant cells which have an improved proliferation potency and antibody-producing efficiency and yet retain the same biological and physical properties as the parent cells.

The monoclonal antibody of the present invention can be isolated and purified from the culture supernatant which is obtained by culturing in vitro the above-noted cells in a conventional serum-free or serum-containing culture medium for animal cells. The purification may be conducted by subjecting the supernatant to conventional biochemical procedures for protein purification, such as ammonium sulfate precipitation, ion exchange chromatography, gel filtration, and affinity chromatography. The thus purified monoclonal antibody may be formulated in a conventional manner to give a biological preparation.

The biological preparations useful for prophylactic and therapeutic treatment of microbisms may contain the monoclonal antibody of the invention directed to exotoxin A of P. aeruginosa as single active ingredient. However, preparations containing at least two human monoclonal antibodies directed to different epitopes of exotoxin A are preferred. The preparations may additionally contain, if desired, other known antibodies which recognize P. aeruginosa-derived antigens other than exotoxins (e.g., LPS), exoenzymes (e.g., elastase and protease), outer membrane proteins, or some part of endotoxins of P. aeruginosa. Further, the preparations may contain, together with the human monoclonal antibodies of the invention, additional antibodies against bacteria other than P. aeruginosa, virus, fungi, protozoa, and cancer cells.

The antibodies of the present invention belong to IgG class, more particularly, IgG 3 subclass, although not limited thereto.

It has been confirmed by immunological assay, such as immunoblotting using exotoxin A fragment of P. aeruginosa, that the monoclonal antibody of the invention recognizes as an epitope 591-613 amino acid sequence

(Glu-

Gln-Ala-Ile-Ser-Ala-Leu-Pro-Asp-Tyr-Ala-Ser-Gln-Pro-Gly-Lys-

Pro-Pro-Arg-Glu-Asp-Leu-Lys)

5

of exotoxin A of P. aeruginosa and binds thereto. Binding of the antibody to exotoxin A results in neutralization of cytotoxicity, lethal toxicity of exotoxin A, thereby P. aeruginosa infections being recovered.

Binding of monoclonal antibody of the invention to exotoxin A of P. aeruginosa is kept stable at a pH of 3.0, which is confirmed in the following manner. Thus, the antibody of the invention adsorbed to sepharose column to which exotoxin A of P. aeruginosa has been chemically linked is not desorbed by washing with a buffer solution of pH 3.0.

The antibodies of the invention include human antibody, mouse-human chimera antibody, humanized antibody, class-subclass switch antibody, and the like, as far as they possess the afore-mentioned characteristics. Antibodies prepared by DNA recombinant technology are also included in the present invention as well as those prepared by EBV transformation method or EBV hybridoma method.

As stated above, the human monoclonal antibody of the present invention recognizes 591-613 amino acid sequence

(Glu-Gln-Ala-Ile-Ser-Ala-Leu-Pro-Asp-Tyr-Ala-Ser-Gln-Pro-

Gly-Lys-Pro-Pro-Arg-Glu-Asp-Leu-Lys)

of exotoxin A of P. aeruginosa and has a high antibody titer to the antigen. The antibody exhibits excellent therapeutic effect on experimental infection in mice when compared to known monoclonal antibodies. The antibody of the invention is advantageous in that it will bring less side-effect such as anaphylaxis because it is a protein of human origin. In addition, the antibody of the invention will be scarcely contaminated with unknown biohazard when compared with conventional immunoglobulin preparations prepared by the use of blood from unspecified individuals, because of the reason that the former is produced using a specified cell. Additional advantage of the antibody of the invention is that it can be steadily supplied because it is produced in vitro, in large amount, and in high titer.

In an effort to more fully illustrate particular aspects of the present invention, the following detailed examples of the preparation of the human monoclonal antibody of the invention are provided. The examples are representative only and should not be construed as limiting in any respect. In the following examples, determination of antibody titer and antibody production were carried out in the manner as described below.

Commercially available exotoxin A of P. aeruginosa (Swiss Serum Institute, Bern, Switzerland) was employed in the examples, which was isolated from the culture supernatant of exotoxin A-producing strain, P. aeruginosa 103 (ATCC 29260) and purified with an anion exchange resin and hydroxyapatite. The exotoxin A was dissolved in a phosphate buffer (pH 7.2, NaCl (8 g/l), KCl (0.2 g/l), $Na_2HPO_4 . 12H_2O$ (2.99 g/l) and $KH_2PO_4$ (0.2 g/l); hereinafter referred to as PBS) to a concentration of 5 $\mu$g/ml. The solution was dispensed on a 96 well microplate (Falcon #3912) at a rate of 100 $\mu$l/well and incubated at 4°C overnight. After removing exotoxin A from the microplate, PBS solution containing 3% bovine serum albumin (hereinafter referred to as BSA) was added at a rate of 120 $\mu$l/well. Incubation at 37°C for 30 minutes gave an antigen-adsorbed microplate in which non-adsorbed portion of exotoxin A was blocked. This microplate was used in the following steps.

Before assay, the antigen-adsorbed plate was washed three times with 0.05% Tween™ 20-containing PBS (hereinafter referred to as PBST). PBST was then dispensed on the plate at a rate of 50 $\mu$l/well, and samples to be tested (serum or culture supernatant), with or without dilution with PBST, were added at a rate of 50 $\mu$l/well, and incubated at 37°C for 2 hours. After removal of the samples, the plate was washed three times with PBST. Second antibody (100 $\mu$l/well) was added and incubated at the same temperature for 2 hours. For determination of human antibody titer, phosphatase-labelled affinity-purified anti-human IgG antibody (Kirkegaard & Perry Lab. Inc.) diluted 500 - 1,000 times with PBST was used as the second antibody.

For determination of mouse antibody titer, phosphatse-lebelled and affinity-purified anti-mouse immunoglobulin antibody (Kirkegaard & Perry Lab. Inc.) was employed. For mouse IgG and IgM antibody titers, phosphatase-labelled anti-mouse IgG antibody and anti-mouse IgM antibody (Kirkegaard & Perry Lab. Inc.) were used respectively. After removal of the second antibody, the plate was washed three times with PBST and a colouring substrate solution (a solution prepared by dissolving p-nitrophenylphosphate 2-sodium salt (3 mg) in a 10% diethanolamine buffer solution (1 ml) containing $NaN_3$ (0.2 mg/ml), $MgCl_2 . 6H_2O$ (0.1 mg/ml)) is added at a rate of 100 $\mu$l/well and reacted at 37°C. After 30 minutes reaction, $OD_{405}$ was measured by multiscan (Titertek).

Determination of human antibody production by ELISA was conducted as follows.

EP 0 421 382 B1

Affinity-purified anti-human IgG (τ chain specific) antibody (Kirkegaard & Perry Lab. Inc.) was dissolved in PBS at a concentration of 10 μg/ml, and the solution was dispensed on a 96 well microplate at a rate of 100 μl/well and incubated at 37 °C for 2 hours to allow the antibody to adsorb the plate. After removal of the solution, 3% BSA-containing PBS was dispensed on the plate at a rate of 120 μl/well and incubated at 37°C for 30 minutes for the purpose of blocking unadsorbed portion of the antibody. The thus obtained plate was used for quantitative determination of antibody production. Purified human IgG (Miles) was used as a standard for the determination. Procedures for ELISA were as described above.

In the accompanying drawings:

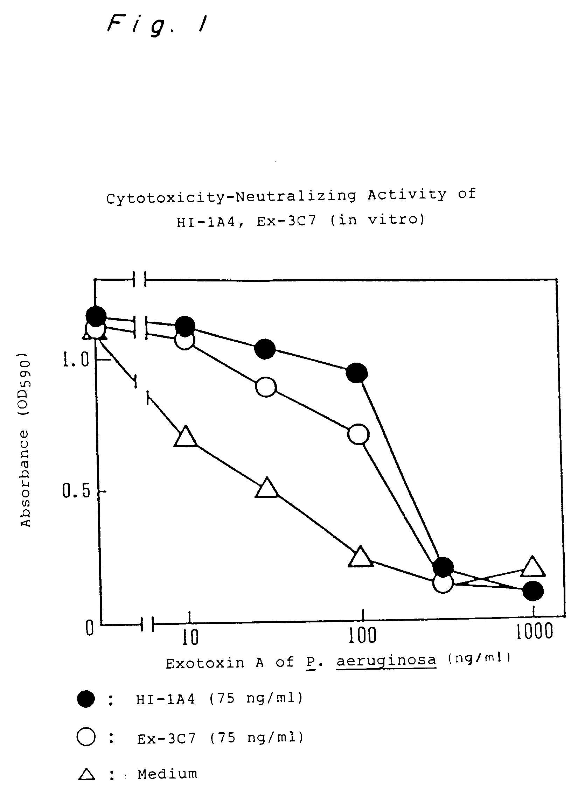

Fig. 1 shows cytotoxicity-neutralizing activities of monoclonal antibodies, HI-1A4 and Ex-3C7. The abscissa shows the amount of exotoxin A of P. aeruginosa and the ordinate shows the absorbance of formazan which generates through decomposition of 3-(4,5-dimethylthiazole-2-yl)-2,5-diphenyl tetrazolium bromide (MTT) by the action of active mitochondrion, which a sign of living cell. The symbols O, O and △ respectively mean HI-1A4, Ex-3C7, and the medium.

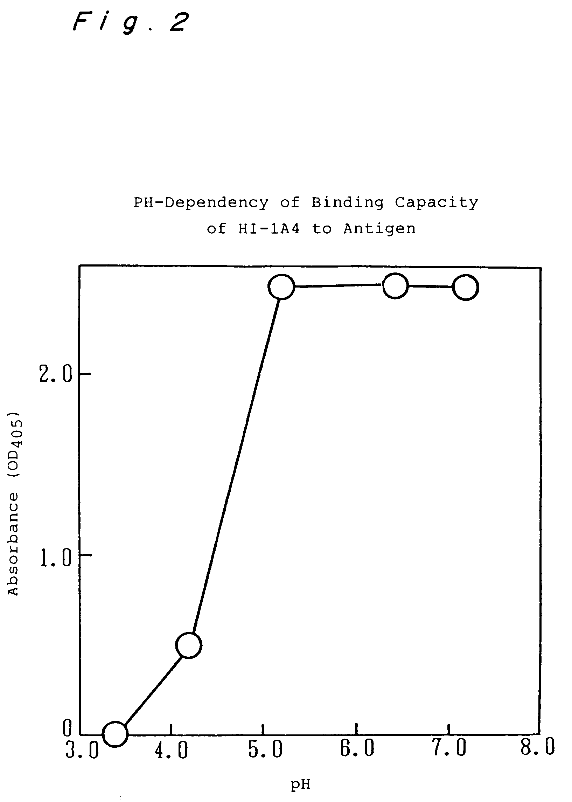

Fig. 2 shows pH-dependency of binding capacity of HI-1A4 to the antigen. The abscissa show pH at which HI-1A4 is allowed to react with the antigen. The ordinate shows the absorbance in ELISA which reflects the amount of HI-1A4 bound to antigen-adsorbed plate.

Example 1 Establishment of human monoclonal antibody-producing clone by EBV transformation method

1. Selection of blood cell donors

Antibody titer to exotoxin A of P. aeruginosa was measured on sera from normal volunteer and P. aeruginosa infected patient. HI, KK and MT who showed high antibody titer were selected as blood cell donors.

2. EBV transformation of human B lymphocytes

(1) Preparation of EBV solution

Marmoset cells B95-8 which produce and release EBV were suspended in Iscove's medium (Boehringer Manheim) containing 15% (v/v) fetal calf serum (FCS)(Hyclone) at a rate of $6.5 \times 10^5$ cells/ml. The suspension in culture flask T-75 (Corning #25110) was stationarily cultured in the presence of 5% $CO_2$ at 37°C for 4 days. The culture supernatant was centrifuged for 10 minutes using a low-speed centrifuge (Tomy-Seiko RS-20BH) at 2,000 rpm (Rotor TS-7). The resultant supernatant was filtered through 0.45 μmø membrane filter (Milex SLHA0250S). The filtrate was charged in Ceram tube (Sumitomo Bakelite MS-4505), stored at 4°C, and used for EBV transformation of human B lymphocytes.

(2) Preparation of lymphocytes from human peripheral blood

Peripheral blood each 20 ml was collected from HI, KK, and MT whose antibody titers to exotoxin A of P. aeruginosa were high. Monopoly separation liquid 15 ml (Flow lab.) was charged in a centrifuge tube (Sumitomo Bakelite, 50 ml volume) and the peripheral blood (20 ml) was gently overlaid thereon. Erythrocytes and lymphocytes were separated by centrifuging with a low speed centrifuge (Tomy-Seiko BS-20BH) under 2,500 rpm (Rotor TS-7) at room temperature for 15 minutes. The layer containing lymphocytes was recovered and washed two times with 15% FCS-containing Iscove's medium. The number of lymphocyte cells counted was $1-3 \times 10^7$ in each donors.

(3) Establishment of anti-exotoxin A antibody-producing cell by EBV transformation method

Human peripheral lymphocytes ($1 \times 10^7$) were suspended in 15% FCS-containing Iscove's medium (2 ml). To the suspension was added afore-mentioned EBV solution (10 ml), and the mixture was incubated in the presence of 5% $CO_2$ at 37°C for 2 hours. EBV-infected human lymphocytes were suspended in 15% FCS-containing Iscove's medium to which cyclosporin A (2 μg/ml) had been added. The resultant suspension (about $2 \times 10^5$ cells/ml) was dispensed on a 96 well culture plate at a rate of 0.1 ml/well. Mouse peritoneal cells (about $1 \times 10^4$ cells/well) were added thereto as protective cells, when necessary. After 4 days culture, the above-mentioned medium (0.1 ml/well) was added, and thereafter, half of the culture medium was replaced by fresh medium every 3 days.

7

After 10-21 days, cell growth was observed in each well. Cells capable of producing specific antibody to exotoxin A of P. aeruginosa were selected by ELISA (Table 1).

Table 1

| Human Monoclonal Antibody to Exotoxin A of P. aeruginosa | |
|---|---|
| Cell | Antibody Titer in Culture Supernatant (ELISA $OD_{405}$) |
| KK-3C3 | >2.0 |
| KK-3C2 | >2.0 |
| KK-1B7 | 2.0 |
| MT-9A5 | >2.0 |
| HI-6E1 | >2.0 |
| HI-1A4 | >2.0 |

(3) Competitive assay in ELISA

Antigen-adsorbed plate was washed three times with PBST. Culture supernatant and exotoxin A of P. aeruginosa as a competitor were dispensed on the plate at a rate of 50 $\mu$l/well and incubated at 37°C for 2 hours. Reaction with second antibody and subsequent manipulation were conducted in accordance with conventional ELISA procedures. The assay results are shown in Table 2.

Table 2

| Competitive Assay in ELISA | | |
|---|---|---|
| Monoclonal Antibody | ELISA Antibody Titer ($OD_{405}$) Exotoxin A of P. aeruginosa ($\mu$g/ml) | |
| | 0 | 5 |
| KK-3C3 | 1.6 | 0.3 |
| KK-3C2 | 1.6 | 0.3 |
| KK-1B7 | 1.8 | 0.1 |
| MT-9A5 | 1.6 | 0.5 |
| HI-6E1 | >2.0 | 0 |
| HI-1A4 | >2.0 | 0 |

Binding of HI-6E1 and HI-1A4 to the antigen adsorbed to the solid phase was significantly inhibited by the addition of exotoxin A of P. aeruginosa, which indicated that these antibodies had an intense binding property to the exotoxin A.

(4) Determination of cytotoxicity-neutralizing activity by cytotoxicity-neutralizing assay using culture cells

Cytotoxicity-neutralizing activity of monoclonal antibody of the invention was determined on the basis of protein-synthetic ability possessed by culture cells. Mouse BALB/c 3T3 cells (2 x $10^5$ cells/well) were suspended in 15% FCS-containing Iscove's medium. The suspension was dispensed on 96 well microplate (Sumitomo Bakelite, MS-3096F) at a rate of 50 $\mu$l/well and incubated overnight at 37°C under 5% $CO_2$.

Exotoxin A of P. aeruginosa was dissolved in the same medium as above at a concentration of 10 ng/ml to prepare exotoxin A solution. Culture supernatant containing 0.1 $\mu$g/ml of human monoclonal antibody to exotoxin A of P. aeruginosa (2 vol) was mixed with exotoxin A solution (1 vol). The mixture was kept at room temperature for 30 minutes to allow neutralizing reaction and then added to the above-mentioned BALB/c 3T3 cells at a rate of 150 $\mu$l/well. After culture at 37°C under 5% $CO_2$ for 72 hours, the culture supernatant was removed. Fixing solution (10% formalin, 100$\mu$l/well) was added, and the plate was allowed to stand at room temperature for 10 minutes. After removal of the fixing solution, a dye solution (0.05% crystal violet, Nakarai Tesk) was added (50 $\mu$l/well). After stationary standing at room temperature for 30 minutes, the fixing solution was removed, and the plate was washed three times with distilled water (250 $\mu$l/well).

Extraction solution (1% acetic acid, 50% methanol) was added at a rate of 100 $\mu$l/well. After five minutes agitation, $CD_{590}$ of the extract was measured. The above test was repeated without addition of exotoxin A as a control assay. Percentage of survived cell number in the test when compared with that of the control was calculated (Table 3).

Table 3

| Cytotoxicity-neutrallizing Activity of Human Monoclonal Antibody in vitro | |
|---|---|
| Monoclonal Antibody | Survived Cell Number (%) |
| KK-3C3 | 24 |
| KK-1B7 | 30 |
| HI-6E1 | 32 |
| HI-1A4 | 100 |

Table 3 shows potent neutralizing activity of HI-1A4.

(5) Cloning of EBV-transformed cell HI-1A4

HI-1A4 (1,000 cells/ml) was suspended in 15% FCS-containing Iscove's medium to which human IL-6 (200 U/ml) had been added. The suspension was dispensed and cloned on a 96 well microplate which contained mouse peritoneal cells (3 x $10^4$ cells/well) at a rate of 0.1 ml/well. After 5 days culture, half of the culture medium was replaced with fresh medium containing human IL-6 (200 U/ml) every 3 days. About half of the wells showed cell growth after two weeks. Culture supernatant of four wells contained specific human antibody against P. aeruginosa. Extensive cultibation of the positive cells was conducted, and cell line HI-1A4-D1 capable of steadily producing comparatively abundant antibody was established. Antibody production of the established cell line was 7 $\mu$g/$10^6$ cells/day. Its doubling time was 48 hours. The cell line continuously produced the antibody for more than 6 months. The cell line showed, when it was cultured in serum-free medium, Cellgrosser-H (Sumitomo Pharmaceuticals), the same magnitude of antibody production as that obtained where the serum-containing medium was employed.

The thus obtained EBV-transformed cell line HI-1A4 has been deposited at the Fermentation Research Institute in Japan under Budapest Treaty and assigned Bikoken Joki No. 3022 (FERM BP-11005). IgG subclass of the antibody produced by HI-1A4 was found to be classified to IgG 3 by ELISA using Human Immunoglobulin G subclass Enzyme Immunoassay (EIA) Kit (Binding Site).

Example 2 Cell fusion

Mouse myeloma cell P3X63Ag8.653 (ATCC No. CRL1580) was subcultured in 15% FCS-containing Iscove's medium. The subcloned cells (2.0 x $10^7$ cells) were washed two times with serum-free Iscove's medium [hereinafter referred to as Iscove's medium). On the other hand, EBV-transformed cell HI-1A4-D1 obtained in Example 1 (2.0 x $10^7$ cells) was washed two times with Iscove's medium. Both cells were fused. Centrifugation gave the fused cells as a precipitate. To the precipitate in the centrifuge tube, 1 ml of polyethylene glycol (PEG) solution (0.45 g PEG 4000, Merck, 0.45 ml PBS(-)) was added over one minute while turning the tube, and then the tube was allowed to stand at room temperature for one minute. One ml of Iscove's medium was added over one minute while turning the tube. This addition was repeated two times. Further, 7 ml of the medium was added over 4 minutes while turning the tube, and the tube was allowed to stand at 37°C for 20 minutes. Centrifugation gave a precipitate, which was suspended in a medium (50 ml) containing 15% FCS, 5 x $10^{-5}$M 2-mercaptoethanol, 100 $\mu$M hypoxanthine, 1$\mu$g/ml azaserine, $10^{-6}$M ouabain, and 5% hybridoma cloning factor (hereinafter referred to as HAz selection medium). The suspension (100 $\mu$l) was dispensed on a 96 well microplate (Falcon #3040) at a rate of 4.0 x $10^4$ cells/well. The cells in microplate was cultured at 37°C under 5% $CO_2$, while half of the culture medium was replaced with fresh HAz selection medium every 2-3 days. The replacement of half medium was conducted every one week by the use of H medium which corresponds to HAz selection medium but lacks azaserine and ouabain. Thereafter, the replacement was conducted every 2-3 days with hypoxanthin-free medium. Culture supernatant from the wells which showed cell growth 3 weeks after the cell fusion was measured by ELISA for specific antibody titer to exotoxin A of P. aeruginosa. Growth of fused cells was detected on 26 wells, while production of specific antibody was observed on all of the wells. Two of 26 fused cells, HI-1A4-5B9 and HI-1A4-5F8 showed steady proliferation and production of specific antibody. Through the cloning of HI-1A4-5B9 by limiting dilution method, we established HI-1A4-6G8 which have antibody-productivity of 30 $\mu$g/ml and doubling time of 35 hours. The established cell line HI-1A4-6G8 has been deposited at the Fermentation Research Institute in Japan under Budapest Treaty and assigned Bikoken Joki No. 3031 (FERM BP-3031) as an accession number.

Example 3 Purification of HI-1A4

After ultra-condensation of HI-1A4 culture supernatant by the use of serum-free medium, monoclonal antibody HI-1A4 was purified by ion exchange chromatography and gel filtration.

The culture supernatant obtained from HI-1A4 culture which was conducted using serum-free Cellgrosser H medium (Sumitomo Pharmaceuticals) was concentrated 84-fold by the use of ultracondensing film YM30 (Amicon Grace Japan). The culture supernatant was charged in an ion-exchange column ABx (J. M. Baker, Inc.), washed with 10 mM phosphate buffer (pH 6.0), and eluted and fractionated under gradient with 0.5 M phosphate buffer (pH 6.8). Fractions containing antibodies which bind to exotoxin A of P. aeruginosa were selected by ELISA, concentrated 8-fold using YM30, and charged in gel filtration column, Superose™ 6 (Pharmacia). The column was eluted with PBS(-), and resultant fractions were subjected to SDS-PAGE, thereby human IgG-containing fractions were selected. About 300 ml of the supernatant gave 1 mg of purified HI-1A4.

Example 4 Comparison of HI-1A4 and mouse monoclonal antibody with respect to neutralizing activity

1. Preparation of mouse monoclonal antibody

(1) Preparation of antigen

Commercially-available exotoxin of P. aeruginosa was dialyzed against PBS and used as immunogen.

(2) Preparation of immunized spleen cells

BALB/c mice (female, 4 week, about 15 g) were subjected to priming by subcutaneous injection of 0.2 ml/mouse of a solution containing 0.5 $\mu$g of exotoxin A of P. aeruginosa, which had been prepared by mixing 5 $\mu$g/ml exotoxin A-containing PBS (one volume) with Freund's complete adjuvant (one volume). Subsequently, booster was conducted four times with two weeks interval using exotoxin A of P. aeruginosa mixed with Freund's incomplete adjuvant, each subcutaneous dose being 2 $\mu$g, 10 $\mu$g, 20 $\mu$g and 20 $\mu$g respectively. About one month after the last booster, the final immunization was conducted by intravenously administering 20 $\mu$g of exotoxin A of P. aeruginosa which had been denatured by repeated freeze-thawing 20 times. Three days after the final immunization, spleen was enucleated, and spleen cells suspended in Eagle's MEM medium (Nakarai Tesk) were prepared. In some trials, fifth booster was additionally conducted one month after the fourth booster by subcutaneous injection of 20 $\mu$g of exotoxin A of P. aeruginosa mixed with Freund's incomplete adjuvant, which made a total of five boosters.

(3) Cell fusion

Mouse myeloma cells (P3X63-Ag8-653, ATCC CRL1580) were cultured in RPMI-1640 medium (Nakarai Tesk) added with 10% fetal calf serum (FCS) (Gibco). The cultured cells were collected at logarithmic phase and used for cell fusion. The cell fusion was conducted in accordance with Kohler's method (Nature, 256 pp495-497, 1975). That is, the spleen cells and myeloma cells (5:1) were suspended in serum-free RPMI-1640 medium and centrifuged at 1,000 rpm (Tomy Seiko CD-100R) for 7 minutes. To the resultant precipitates was added over one minute a 10% (v/v) dimethylsulfoxide solution (1 ml) containing 45% (w/w) polyethylene glycol (PEG) 4000 warmed at 37°C, and the mixture was allowed to stand at 37°C for one minute. To the mixture was added 2 ml of serum-free RPMI-1640 medium at a rate of 1 ml/min. Additional 7 ml of the serum-free RPMI-1640 medium was added thereto, thereby PEG being diluted. The mixture was allowed to stand for 20 minutes at 37°C and centrifuged at 1,000 rpm for 7 minutes. The resultant precipitate was resuspended in 10% FCS-containing RPMI1640 medium so that a concentration of $6 \times 10^4$ cells/ml may be obtained, and the suspension was dispensed on 96 well microplate (Sumitomo Bakelite MS-3096F) at a rate of 0.2 ml/well. The fused cells were cultured at 37°C under 5% $CO_2$.

One day after cell fusion, half of the culture medium was replaced by fresh HAT medium (RPMI1640 medium containing $10^{-4}$ M hypoxanthine, $4 \times 10^{-7}$ M aminopterin, $1.6 \times 10^{-5}$ M thymidine). Thereafter, replacement of half of the culture medium with HAT medium was conducted three times every two days. After ten days culture, 70% wells showed cell growth. ELISA revealed that 42 wells produced specific antibody. Antibody titers of the culture supernatant obtained from representative positive wells are shown in Table 4.

Table 4

| Mouse Monoclonal Antibody to Exotoxin A of P. aeruginosa | |
| --- | --- |
| Antibody-Producing Cell | ELISA Antibody Titer (OD$_{405}$) |
| Ex-1F2 | 0.7 |
| Ex-2A10 | 1.2 |
| Ex-2E1 | 0.3 |
| Ex-2H4 | >2.0 |
| Ex-3C7 | >2.0 |
| Ex-3D5 | 1.4 |
| Ex-3G8 | >2.0 |
| Ex-4A7 | >2.0 |
| Ex-4E2 | 1.3 |
| Ex-4F2 | >2.0 |
| Ex-5A10 | 0.8 |
| Ex-5C11 | >2.0 |
| Ex-5D4 | 0.9 |
| Ex-6C12 | 1.0 |
| Ex-7D10 | 0.3 |
| Ex-7D2 | 1.8 |
| Ex-7G2 | 1.8 |
| Ex-8B5 | 0.5 |
| Ex-8H5 | >2.0 |
| Ex-9E1 | 2.0 |

(4) Preparation of ascites fluid

BALB/c mice received pristane (2,6,10,14-tetramethylpentadecane; Aldrich Chemical Co., Inc.) at a rate of 0.5 ml/mouse. After 4 weeks, $5 \times 10^6$ hybridoma obtained by culturing in a test tube after cloning were intraperitoneally administered to the mice, and ascites fluid was taken from peritoneal after 7 - 14 days.

(5) Purification

The mouse ascites fluid was purified using Protein A monoclonal antibody purification system (MAPS, Bio-Rad Lab.). That is, one part of the ascites fluid was mixed with one part of a binding buffer (pH 9.0), which was attached to the above-mentioned system, and the mixture was applied to affigel Protein A column. The column was subjected to elution, after washing with the binding buffer, with an eluting buffer (pH 3.0), and the eluate was immediately dialyzed against PBS. Antibody titer was measured by protein assay kit I (Bio-Rad).

2. Determination of cytotoxicity-neutralizing activity

Mouse BALB/c 3T3 cells were suspended in 15% FCS-containing Iscove's medium at a rate of $2 \times 10^5$ cells/ml, and the suspension was dispensed on 96 well microplate (Sumitomo Bakelite MS-3096F) at a rate of 100 μl/well and cultured overnight at 37°C under 5% $CO_2$.

Ascites fluid containing mouse monoclonal antibody to exotoxin A of P. aeruginosa was diluted 50-fold with 10% FCS-containing DMEM medium to obtain an antibody solution. On the other hand, exotoxin A of P. aeruginosa was dissolved in 10% FCS-containing DEME medium at a concentration of 0.1 ng/ml to obtain an exotoxin A solution. The antibody solution was mixed with an equal amount of the exotoxin A solution. Neutralizing reaction was allowed to proceed at room temperature for 30 minutes, and the mixture was added to the afore-mentioned BALB/c 3T3 cells at a rate of 100 $\mu$l/well. After 14-hour culture at 37°C under 5% $CO_2$, 100 $\mu$l of the culture supernatant was removed, and additional 2-hour culture was conducted with the addition of $^3H$ leucine (0.25 $\mu$Ci/well; 1 Ci/mg; Amersham Corporation). The cells were washed three times with PBS, and scraped off from the well with 0.1 N NaOH. The resultant floating cells were incubated with 10% trichloroacetic acid (TCA), which had been previously cooled to 4°C, at 4°C for 30 minutes. The amount of $^3H$ taken in TCA insoluble fractions was measured by liquid scintillation counter (Beckman). Antibodies which neutralized more than 50% of inhibition of protein synthesis caused by exotoxin A of P. aeruginosa were evaluated as positive with respect to neutralizing activity. The test results are shown in Table 5.

Table 5

| Cytotoxicity-neutralizing Activity of Mouse Monoclonal Antibody (Qualitative Assay) | |
| --- | --- |
| Monoclonal Antibody | Cytotoxicity-Neutralizing Activity |
| Ex-1F2 | - |
| Ex-2A10 | - |
| Ex-2H4 | - |
| Ex-3C7 | + |
| Ex-3D5 | - |
| Ex-3G8 | - |
| Ex-3H4 | - |
| Ex-4A7 | - |
| Ex-4E2 | - |
| Ex-4F2 | + |
| Ex-5A10 | - |
| Ex-5C11 | - |
| Ex-5D4 | - |
| Ex-6C12 | - |
| Ex-7D10 | - |
| Ex-8H5 | + |

Mouse cells Ex-3C7, Ex-4F2, and Ex-8H5 showed neutralizing effect on cytotoxicity caused by exotoxin A of P. aeruginosa.

Cytotoxicity-neutralizing activity of purified monoclonal antibody was measured using the method just mentioned above. The results are shown in Table 6.

Table 6

| Cytotoxicity-neutralizing Activity of Representative Mouse Monoclonal Antibodies | |
| --- | --- |
| Monoclonal Antibody | Cytotoxicity-Neutralizing Activity (%) |
| Ex-2A10 | 0 |
| Ex-3C7 | 73 |
| Ex-4F2 | 30 |
| Ex-8H5 | 30 |

Ex-3C7 showed potent neutralizing activity. While Ex-4F2 and Ex-8H5 also showed the activity, Ex-2A10 showed no activity.

3. Neutralizing activity on cytotoxicity caused by exotoxin A of P. aeruginosa (in vivo)

Purified mouse monoclonal antibody to exotoxin A of P. aeruginosa was intraperitoneally administered to ICR-slc mice (4 weeks old, male, 10 mice/group) at a rate of 20 $\mu$g/mouse. For control, only 5 mg of BSA

12

was administered. After one hour, 0.8 $\mu$g of exotoxin A of P. aeruginosa was intraperitoneally injected. Survival ratio of mice on 7th day after inoculation of exotoxin A are shown in Table 7.

Table 7

| Survival Ratio on 7th Day After Inoculation of Exotoxin A | |
|---|---|
| Monoclonal Antibody | Survival Ratio (%) |
| Ex-2A10 | 10 |
| Ex-3C7 | 80 |
| Ex-4F2 | 50 |
| Ex-8H5 | 30 |
| BSA | 10 |

Monoclonal Ex-3C7 showed stronger neutralizing activity in vivo than Ex-4F2 and Ex-8H5, which indicated that Ex-3C7 had the strongest cytotoxicity-neutralizing activity among mouse monoclonal antibodies.

4. Comparison of cytotoxicity-neutralizing activity using cultured cells

Human BALL 1 cells were suspended (5 x 10$^5$ cells/ml) in 15% FCS-containing Iscove's medium, and the suspension was dispensed on 96 well microplate (Sumitomo Bakelite, MS-3096F) at a rate of 100 $\mu$l/well.

Monoclonal antibody to exotoxin A of P. aeruginosa was diluted with the same medium to a concentration of 0.3 $\mu$g/ml to obtain an antibody solution, while exotoxin A of P. aeruginosa was diluted with the same medium to a concentration of 0.04 - 4 $\mu$g/ml to obtain an exotoxin A solution. Each of the solutions was added to the above BALL 1 cells at a rate of 50 $\mu$l/well. The cells were cultured at 37°C under 5% CO$_2$ for 5 days, and living cells were quantitatively measured by MTT cell growth assay (Chemicon International Inc.). The test results are shown in Fig. 1 of the accompanying drawings. HI-1A4 showed exotoxin A-neutralizing activity stronger by several times than Ex-3C7. One molecule of the former neutralized more than one molecule of exotoxin A.

5. Neutralizing activity on cytotoxicity caused by exotoxin A of P. aeruginosa (in vivo) - therapeutic effect

Monoclonal Antibodies to exotoxin A of P. aeruginosa, HI-1A4 (0.31 - 3.1 $\mu$g/mouse) and Ex-3C7 (0.43 - 4.3 $\mu$g/mouse), were intraperitoneally administered to ICR-slc mice (4 weeks, male, 10 mice/ group). For control, only BSA (10 mg/mouse) was administered. After one hour, 1 $\mu$g of exotoxin A of P. aeruginosa was intraperitoneally administered. Survival ratio of mice on 7th day after inoculation of exotoxin A is shown in Table 8.

13

Table 8

| Anti-exotoxin A of P. aeruginosa Activity (in vivo) | | | |
|---|---|---|---|
| Antibody | Dose ($\mu$g/head) | Survival Ratio (%) | $ED_{50}$ ($\mu$g/head) |
| HI-1A4 | 0.31<br>0.63<br>1.3<br>3.1 | 10<br>80<br>70<br>100 | 0.58 |
| Ex-3C7 | 0.43<br>0.85<br>1.7<br>4.3 | 20<br>20<br>70<br>100 | 1.1 |
| BSA<br>non treated | 10 | 20<br>10 | |

Human monoclonal antibody HI-1A4 showed neutralizing activity about 2-fold stronger than mouse monoclonal antibody Ex-3C7.

6. Determination of enzyme-neutralizing activity

Further study was conducted to determine if anti-Ex-A-monoclonal antibody neutralizes Ex-A's enzymatic activity, i.e., ADP ribosyltransferase (hereinafter referred to as ADPRTase) K activity against elongation factor 2 (hereinafter referred to as EF2).

(1) Purification of EF2

EF2 was purified in accordance with K. Takamatsu et al method (Biochemical and Biophysical Research Communication, 134, pp1015-1021, 1986). That is, livers taken from 60 rats were homogenized at 0 - 4°C by Waring blender together with 5 volumes of cooled 0.25 M sucrose solution containing 3.3 mM phenylmethylsulfonyl fluoride. After filtration of tissue debris with Nylon mesh, the homogenate was centrifuged at 16,000 x g for 20 minutes. To the supernatant was added dithiothreitol (DTT) at a concentration of 1.5 mM, and the mixture was subjected to fractional precipitation by addition of 30 - 65% saturated ammonium sulfate. The precipitate dissolved in Buffer A (50 mM Tris-HCl buffer, pH 8.3, 2.5 mM DTT, 5% glycerol) was dialyzed against Buffer A. Dialysate was applied to DE-52 column. After washing with Buffer A, the column was eluted with linear gradient of Buffer A containing 0 - 2.5 M NaCl to obtain crude fraction EF2. To the crude fraction (3.5 ml) were added 0.5 ml of 0.1 M Tris-HCl buffer, pH 7.9, 1.0 ml of 0.5 M DTT, 2.0 ml of 50% glycerol, and 3.0 ml of 10 mg/ml calf serum albumin, and the mixture was stored at -20°C.

(2) Activation of Ex-A

Ex-A (0.5 mg/ml) was activated through incubation in the presence of 4 M urea, and 1% (w/v) DTT at 22°C for 15 minutes.

(3) Determination of ADPRTase activity

ADPRTase activity was measured in accordance with M. Pollack's method (J. Infect. Dis. 145 pp688-698, 1982). Thus, a mixture of 50 $\mu$l of EF-II solution which had been prepared by adding 30 $\mu$l of $^{14}$C-NAD$^+$ (NEN, 450 mCi/mm, 1.22 $\mu$M) and 2 $\mu$l of 250 mM EDTA to 500 $\mu$l of EF-II solution obtained in (1) above, 600 ng of purified monoclonal antibody, and 5 $\mu$l of a solution containing 50 ng of Ex-A activated in (2) above, pre-incubated at room temperature for 30 minutes, was allowed to react. After 5 minutes at room temperature, 55 $\mu$l of cooled 10% TCA was added thereto, and the mixture was allowed to stand on ice for 10 minutes. TCA-insoluble fractions were collected on a glass filter (Whatman, GF/C), and the amount of intaken $^{14}$C was measured by liquid scintillation counter (Beckman). The results are shown in Table 9.

Table 9   Enzymatic Activity-neutralizing Activity of
          Purified Antibody

| Monoclonal Antibody | Neutralizing Activity (%)[*1] |
|---|---|
| HI-1A4 | 100 |
| Ex-2A10 | 45.2 |
| Ex-3C7 | 6.6 |
| Ex-4F2 | 20.6 |

*1   Activity is shown in percentage (%) calculated
     in accordance with the following equation.

$$\left[1-\frac{\text{Antibody added (cpm) - Background (cpm)}}{\text{Antibody not added (cpm) - Background (cpm)}}\right] \times 100$$

Monoclonal antibodies HI-1A4 and Ex-2A10 inhibited EFII ADP ribosylation. HI-1A4 showed stronger neutralizing activity than mouse monoclonal antibody Ex-2A10.

7. Therapeutic effect of monoclonal antibody on subcutaneous infection of P. aeruginosa in mice

ICR-slc mice (4 weeks old, male, ten mice/group) received 0.2 ml ($3 \times 10^7$ CFU) of a suspension of P. aeruginosa SP9791b, which produces exotoxins, by subcutaneous inoculation on the back. Two hours after inoculation, HI-1A4 (0.19-6.3 μg/head) or Ex-3C7 (0.26-8.5 μg/head) dissolved in PBS was intravenously injected. Twenty-four hours after inoculation, gentamycin (4 mg/head) was subcutaneously and intraperitoneally administered each by half. For control, only BSA was administered. Therapeutic effect of antibodies was determined on the basis of survival ratio on 7th day after administration of the antibodies. The results are shown in Table 10.

Table 10

| Therapeutic Effect of Antibody on P. aeruginosa Infected Mouse | | | | |
|---|---|---|---|---|
| Antibody | Dose (μg/head) | Gentamycin | Survival Ratio(%) | $ED_{50}$ (μg/head) |
| HI-1A4 | 0.19 | + | 0 | |
| | 0.63 | + | 40 | 1.1 |
| | 1.9 | + | 80 | |
| | 6.3 | + | 80 | |
| | 6.3 | - | 30 | |
| Ex-3C7 | 0.26 | + | 10 | |
| | 0.85 | + | 0 | 1.8 |
| | 2.6 | + | 80 | |
| | 8.5 | + | 90 | |
| | 8.5 | - | 50 | |
| BSA | 10 | + | 0 | |
| | 10 | - | 0 | |
| not administered | - | + | 0 | |
| | - | - | 0 | |

HI-1A4, when administered together with gentamycin, showed strong therapeutic effect on infections. The therapeutic effect of HI-1A4 was equal to about doubled amount of Ex-3C7. HI-1A4 also showed therapeutic effect even when administered alone, which had never been observed in conventional IgG antibodies.

Example 5 Study on properties of human monoclonal antibody HI-1A4

1. Identification of epitope

(1) Fragmentation of exotoxin A molecule of P. aeruginosa and reactivity against the antibody

i) Reactivity against chymotrypsin-treated fragment 26K

Ex-A was dissolved in 10 mM Tris-HCl buffer (pH 8.0) at a concentration of 2 ng/ml, and the solution was admixed with the same amount of 8 M aqueous urea solution. To the mixture was added dithiothreitol to the final concentration of 5 mM, and protein was denatured at room temperature for 20 minutes. The reaction mixture was subjected to gel-filtration using PD-10 column so that the buffer may be changed to 1 mM EDTA-containing 50 mM Tris-HCl buffer (pH 8.2). After protection of NAD-binding site by addition of NAD at the final concentration of 10 mM, the protein was reacted with chymotrypsin (100 μg/ml) at 25°C for 90 minutes to obtain fragments. The reaction was terminated by addition of 10 μg/ml PMSF, and the reaction mixture was subjected to high pressure liquid chromatography for fractionation, thereby a fraction containing a fragment having a molecular weight of 26 K was obtained. The fraction was applied to a peptide-sequencer, which revealed that the fragment corresponded to 591 - 613 amino acids of Ex-A molecule.

On the other hand, the reaction mixture was heated at 100°C for 5 minutes with addition of 2% SDS, 5% 2-mercaptoethanol, and 10% glycerol-containing 125 mM Tris-HCl buffer (pH 6.8), and subjected to SDS/polyacrylamide gel electrophoresis. The gel was dipped in a transfer buffer (10 mM CAPS, 10% (v/v) methanol, pH 11.0) at room temperature for 30 minutes. The gel, after dipping in methanol for one minute, was transferred electrically (50V, 10 hours) at 4°C to polyvinyldifluoride (PVDF) film (millipore) which had been dipped in a transfer buffer for 30 minutes. Blocking was achieved by incubation of the film at room temperature for 30 minutes in 2% casein-containing PBS.

Ascites fluid containing Ex-3C7, after dilution 1000-fold with 2% casein-containing PBS, was incubated at room temperature for 2 hours. The PVDF film was washed three times with 0.05% Tween 20-containing PBS, and incubated at 37°C for 2 hours with alkaline phosphatase-labelled anti-mouse immunoglobulin G antibody which was 1000-fold diluted with 2% casein-containing PBS. Similarly, the film was washed five times with 0.05% Tween™ 20-containing PBS and then incubated with addition of 10% diethanolamine buffer (pH 9.1) containing 0.5 mg of a coloring substrate solution (4-bromo-2-chloroindophenol phosphate (1 mg), NaN₃ (0.2 mg/ml), MgCO₂.6H₂O (0.1 mg/ml) for colouring.

Human monoclonal antibody HI-1A4 has bound to chymotrypsin-treated 26K which corresponds to 39 - 613 (C-terminal) amino acids of exotoxin A of P. aeruginosa. This means that the monoclonal antibody of the present invention recognizes C-terminal of exotoxin A of P. aeruginosa.

ii) Reactivity to endopeptidase Lys-C treated fragments 42K, 40K

Ex-A was dissolved in 10 mM Tris-HCl buffer (pH 8.0) at a concentration of 2 mg/ml. The solution was combined with the same amount of 8M aqueous urea solution, and to the resultant mixture was added dithiothreitol to the final concentration of 5 mM. The mixture was kept at room temperature for 20 minutes for denaturation of proteins. After reaction, the buffer was changed to 50 mM Tris-HCl buffer (pH 9.0) by gel-filtration using PD-10 column and the concentration of exotoxin A of P. aeruginosa was made to 0.5 mg/ml. Eendopeptidase Lys C (Boeringer Mannheim) was added thereto at a concentration of 8 µg/ml, and the mixture was allowed to react at 37°C for 2 hours. After addition of the same amount of endopeptidase Lys C, the mixture was allowed to react at 37°C for 2 hours. The reaction was terminated by addition of 5 µg/ml of phenylmethane sulfonyl fluoride (Sigma Chemical Company). The reaction mixture was subjected to SDS electrophoresis and immunoblotting in the same manner as described in (i) above, whereby the investigation was made on the epitope to which HI-1A4 binds.

Cleavage by endopeptidase Lys-C gave 42K and 40K fragments. It was found that 42K fragment corresponded to 234 or 241 - 613 (C-terminal) amino acids of exotoxin A of P. aeruginosa, while 40K fragment corresponded to 241 - 590 amino acids. Immunoblotting revealed that HI-1A4 reacted with 42K band, but not with 40K band. Accordingly, it was found that the epitope of HI-1A4 existed in 591 - 613 amino acids

(Glu-Gln-Ala-Ile-Ser-Ala-Leu-Pro-Asp-Tyr-Ala-Ser-Gln-

Pro-Gly-Lys-Pro-Pro-Arg-Glu-Asp-Leu-Lys).

iii) Determination of epitope by modification of amino acid residue

Tyrosine residue of exotoxin A of P. aeruginosa was modified with iodine, and the reactivity of the modified antigen against HI-1A4 was investigated.

Exotoxin A of P. aeruginosa was dissolved in 5 mM Tris-HCl buffer (pH 8.0) at a concentration of 1 mg/ml. To the solution was added the same volume of an aqueous 0.1 M iodine and 0.1 M KI solution and the mixture was allowed to react for iodination of exotoxin A.

The effect of iodination was investigated by competitive reaction in ELISA, in which the concentration of HI-1A4 was 0.1 µg/ml and the concentration of the competitive substance was 5 µg/ml. The test results are shown in Table 11.

Table 11

| Effect of Chemical Modification of Tyrosine Residue on Reactivity to HI-1A4 | |
| --- | --- |
| Competitive Substance | Inhibition (%) |
| Modified Exotoxin A | 33 |
| Exotoxin A | 92 |
| Mock | 0 |

The table shows that chemical modification of tyrosine reduces by 60% the antigen's reactivity to HI-1A4, which indicates in turn that the tyrosine residue constitutes part of the epitope. Amino acid sequence from position 591 to 613

```
(Glu-Gln-Ala-Ile-Ser-Ala-Leu-Pro-Asp-

Tyr-Ala-Ser-Gln-Pro-Gly-Lys-Pro-Pro-Arg-Glu-Asp-Leu-Lys),
```

in which the epitope exists, contains only one tyrosine residue (position 600). Thus, it was concluded that the epitope of the monoclonal antibody of the present invention locates in the vinicity of tyrosine at position 600.

(2) PH-dependency of the binding of antigen with exotoxin A of P. aeruginosa

PH-dependency of binding of exotoxin A of P. aeruginosa with the human monoclonal antibody of the invention was investigated by changing pH under which the antibody is allowed to bind to the antigen-adsorbed plate in ELISA. The results are shown in Fig. 2 of the accompanying drawings. Fig. 2 shows that HI-1A5 intensively binds to exotoxin A of P. aeruginosa at pH higher than 5, but not at pH 4.

Exotoxin A of P. aeruginosa was allowed to bind to CNBr-sepharose charged in a column to prepare Ex-A-sepharose column. By the use of thus prepared column, a magnitude of resistance of the binding created between HI-1A4 and exotoxin A of P. aeruginosa to lower pH was investigated. Thus, HI-1A4 was adsorbed in the column at a neutral pH. When elution was conducted with an elution buffer at pH 3.0, HI-1A4 was not eluted, which indicated that the binding once created was stable at pH 3.

The fact that the binding of HI-1A4 with exotoxin A of P. aeruginosa is stably retained at lower pH suggests that the exotoxin A-antibody complex is not decomposed during fusion of exotoxin A to lysosome (low pH) and its intracellular transportatior in the fused form, and that the antibody may thus successfully inhibit the expression of toxicity of exotoxin A.

As stated above, the human monoclonal antibody of the invention, HI-1A4, has an excellent characteristics that have never been observed thus far.

**Claims**

1. A human monoclonal antibody having the following characteristics:
   (1) the monoclonal antibody specifically binds to exotoxin A of Pseudomonas aeruginosa;
   (2) one molecule of the monoclonal antibody neutralizes cytotoxicity caused by more than one molecule of exotoxin A of P. aeruginosa in a cell culture system;
   (3) $4 \times 10^{-12}$ mole of the monoclonal antibody parenterally administered to mice neutralizes lethal toxicity caused by $15 \times 10^{-12}$ mole of exotoxin A of P. aeruginosa and keeps more than 50% of mice alive;
   (4) the monoclonal antibody is prophylactically and therapeutically effective for P. aeruginosa infections associated with exotoxin A;
   (5) the monoclonal antibody shows synergistic effects in the treatment of P. aeruginosa infections associated with exotoxin A, when used together with antibiotics;
   (6) the epitope of the monoclonal antibody exists in the 591 - 613 amino acid residues of exotoxin A of P. aeruginosa:

```
Glu-Gln-Ala-Ile-Ser-Ala-Leu-Pro-Asp-Tyr-Ala-

Ser-Gln-Pro-Gly-Lys-Pro-Pro-Arg-Glu-Asp-Leu-Lys
```

   of exotoxin A
   (7) the monoclonal antibody belongs to IgG 3 class;
   (8) the monoclonal antibody exhibits maximum binding activity to exotoxin A of P. aeruginosa at pH higher than 5, and thus binding of the monoclonal antibody to the exotoxin A is kept stable at pH 3.

2. A cell line producing the human monoclonal antibody of Claim 1 and its descendant cell lines.

3. The cell line of Claim 2 which is an EBV-transformed cell line.

4. A fused cell line producing human monoclonal antibody, which is obtained by cell fusion of a myeloma cell line with an EBV-transformed cell line of Claim 3, and its descendant cell lines.

5. EBV-transformed cell line HI-1A4 having the deposit accession number FERM BP-3022.

6. Human monoclonal antibody obtainable from the cell line of Claim 5.

7. Fused cell line HI-1A4-6G8 having the deposit accession number FERM BP-3031.

8. Human monoclonal antibody obtainable from the cell line of Claim 7.

9. A pharmaceutical preparation containing the human monoclonal antibody of Claim 1, 6 or 8.

10. A pharmaceutical preparation for preventing and treating P. aeruginosa infections, which contains as an essential component the human monoclonal antibody of Claim 1, 6 or 8.

11. A process for producing the human monoclonal antibody of Claim 1, which process is characterized by culturing the cell line of Claims 2 to 5 or 7 or descendants thereof.

**Patentansprüche**

1. Monoclonaler menschlicher Antikörper, dadurch gekennzeichnet, daß:
    (1) der monoclonale Antikörper an Exotoxin A von Pseudomonas aeruginosa spezifisch bindet,
    (2) ein Molekül des monoclonalen Antikörpers die durch mehr als ein Molekül Exotoxin A von P. Aeruginosa in einem Zellkultursystem verursachte Cytotoxizität neutralisiert,
    (3) $4 \times 10^{-12}$ Mol des an Mäuse parenteral verabreichten monoclonalen Antikörpers die durch $15 \times 10^{-12}$ Mol Exotoxin A von P. aeruginosa verursachte letale Toxizität neutralisieren und mehr als 50 % der Mäuse überleben,
    (4) der monoclonale Antikörper bei mit Exotoxin A assoziierten P. aeruginosa-Infektionen prophylaktisch und therapeutisch wirksam ist,
    (5) der monoclonale Antikörper bei der Behandlung von mit Exotoxin A assoziierten P. aeruginosa-Infektionen bei Verwendung zusammen mit Antibiotika synergistische Wirkungen zeigt,
    (6) das Epitop des monoclonalen Antikörpers in den Aminosäureresten 591 - 613 von Exotoxin A von P. aeruginosa vorkommt:

$$\text{Glu-Gln-Ala-Ile-Ser-Ala-Leu-}$$
$$\text{Pro-Asp-Tyr-Ala-Ser-Gln-Pro-Gly-Lys-Pro-Pro-Arg-Glu-}$$
$$\text{Asp-Leu-Lys}$$

    von Exotoxin A,
    (7) der monoclonale Antikörper zur Klasse IgG 3 gehört,
    (8) der monoclonale Antikörper maximale Bindungsaktivität an Exotoxin A von P. aeruginosa bei einem pH-Wert über 5 zeigt und daher die Bindung des monoclonalen Antikörpers an das Exotoxin A bei einem pH-Wert von 3 stabil gehalten wird.

2. Zellinie, den menschlichen monoclonalen Antikörper von Anspruch 1 erzeugend, und davon abstammende Zellinien.

3. Zellinie nach Anspruch 2, die eine EBV-transformierte Zellinie ist.

4. Fusionierte, einen menschlichen monoclonalen Antikörper erzeugende Zellinie, erhalten durch Zellfusion einer Myelomzellinie mit einer EBV-transformierten Zellinie von Anspruch 3, und davon abstammende Zellinien.

5. EBV-transformierte Zellinie HI-1A4 mit der Hinterlegungsnummer FERM BP-3022.

**6.** Menschlicher monoclonaler Antikörper, erhältlich von der Zellinie von Anspruch 5.

**7.** Fusionierte Zellinie HI-1A4-6G8 mit der Hinterlegungsnummer FERM BP-3031.

**8.** Menschlicher monoclonaler Antikörper, erhältlich von der Zellinie von Anspruch 7.

**9.** Arzneimittel, den menschlichen monoclonalen Antikörper von Anspruch 1, 6 oder 8 enthaltend.

**10.** Arzneimittel zur Verhütung und zur Behandlung von P. aeruginosa-Infektionen, das als einen wesentlichen Bestandteil den menschlichen monoclonalen Antikörper von Anspruch 1, 6 oder 8 enthält.

**11.** Verfahren zur Herstellung des menschlichen monoclonalen Antikörpers nach Anspruch 1, dadurch gekennzeichnet, daß die Zellinie der Ansprüche 2 bis 5 oder 7 oder deren Nachkommen gezüchtet werden.

**Revendications**

**1.** Anticorps monoclonal humain ayant les caractéristiques suivantes:
(1) l'anticorps monoclonal se lie de façon spécifique à l'exotoxine A de *Pseudomonas aeruginosa*;
(2) une molécule de l'anticorps monoclonal neutralise la cytotoxicité provoquée par plus d'une molécule d'exotoxine A de *P. aeruginosa* dans un système de culture cellulaire;
(3) l'administration parentérale de $4 \times 10^{-12}$ mole de l'anticorps monoclonal à des souris permet de neutraliser la toxicité létale entraînée $15 \times 10^{-12}$ mole d'exotoxine A de *P. aeruginosa* et maintient en vie plus de 50 % des souris;
(4) l'anticorps monoclonal a un effet prophylactique et thérapeutique contre les infections à *P. aeruginosa* associées à l'exotoxine A;
(5) l'anticorps monoclonal présente des effets synergiques dans le traitement des infections à *P. aeruginosa* associées à l'exotoxine A, lorsqu'il est utilisé avec des antibiotiques;
(6) l'épitope de l'anticorps monoclonal se trouve dans les résidus 591 - 613 de l'exotoxine A de *P. aeruginosa*:

Glu-Gln-Ala-Ile-Ser-Ala-Leu-Pro-Asp-
Tyr-Ala-Ser-Gln-Pro-Gly-Lys-Pro-Pro-Arg-Glu-Asp-Leu-Lys-

de l'exotoxine A;
(7) l'anticorps monoclonal appartient à la classe des IgG 3;
(8) l'anticorps monoclonal manifeste une activité de liaison maximale à l'exotoxine A de *P. aeruginosa* à un pH supérieur à 5, et cette liaison de l'anticorps monoclonal à l'exotoxine A reste stable à pH 3.

**2.** Lignée cellulaire produisant un anticorps monoclonal humain selon la revendication 1 et ses liées cellulaires descendantes.

**3.** Lignée cellulaire selon la revendication 2, qui est une lignée cellulaire transformée avec l'EBV.

**4.** Lignée cellulaire fusionnée produisant un anticorps monoclonal humain, qui est obtenue par fusion cellulaire d'une lignée cellulaire de myélome avec une lignée cellulaire transformée avec l'EBV selon la revendication 3, et ses lignées cellulaires descendantes.

**5.** Lignée cellulaire HI-1A4 transformée avec l'EBV, déposée sous le numéro d'accès FERM BP-3022.

**6.** Anticorps monoclonal humain pouvant être obtenu à partir de la lignée cellulaire de la revendication 5.

**7.** Lignée cellulaire fusionnée HI-1A4-6G8 déposée sous le numéro d'accès FERM BP-3031.

**8.** Anticorps monoclonal humain pouvant être obtenu à partir de la lignée cellulaire de la revendication 7.

9. Préparation pharmaceutique contenant un anticorps monoclonal humain selon la revendication 1, 6 ou 8.

10. Préparation pharmaceutique pour la prévention et le traitement d'infections à *P. aeruginosa*, qui contient comme constituant essentiel un anticorps monoclonal humain selon la revendication 1, 6 ou 8.

11. Procédé de production d'un anticorps monoclonal humain selon la revendication 1, ce procédé étant caractérisé par la culture d'une lignée cellulaire selon les revendications 2 à 5 ou 7 ou de ses descendants.

*Fig. 1*

Cytotoxicity-Neutralizing Activity of
HI-1A4, Ex-3C7 (in vitro)

● : HI-1A4 (75 ng/ml)

○ : Ex-3C7 (75 ng/ml)

△ : Medium

*Fig. 2*

PH-Dependency of Binding Capacity
of HI-1A4 to Antigen